# EUROPEAN PATENT SPECIFICATION

(11) **EP 1 381 627 B1**
(45) Date of publication and mention of the grant of the patent: **10.11.2010**
(21) Application number: 02720228.2
(22) Date of filing: 17.04.2002
(51) Int. Cl.: C07K 14/47, C07K 5/09, C07K 5/11, C07K 5/103, A61K 38/17, A61P 25/28

(54) **POLYPEPTIDES DERIVED FROM AMYLOID PRECURSOR PEPTIDE (APP) AND THEIR USES**
POLYPEPTIDE, ABGELEITET VON DER AMYLOID VORLÄUFERPROTEIN (APP), UND IHRE VERWENDUNGEN
POLYPEPTIDES DERIVES DU PRECURSEUR DE LA PROTEINE AMYLOIDE (AAP), ET LEUR UTILISATION

(30) Priority: 18.04.2001 GB 0109558; 17.08.2001 GB 0120084; 30.11.2001 US 998491; 28.03.2002 GB 0207387
(43) Date of publication of application: 21.01.2004
(60) Divisional application: 10154130.8
(73) Proprietor: THE OPEN UNIVERSITY, Milton Keynes, Buckinghamshire MK7 6BJ (GB)
(72) Inventor: MILEUSNIC, Radmila, c/o Dept. of Biological Sciences, Milton Keynes MK7 6AA (GB); ROSE, Steven Peter Russell, c/o Dept. of Biological Sciences, Milton Keynes MK7 6AA (GB)
(74) Representative: W.P. Thompson & Co.
(86) International application number: PCT/GB2002/001769
(87) International publication number: WO 2002/083729

(56) References cited:
- WO-A-94/09808
- WO-A-97/00063
- WO-A-97/04748
- WO-A-98/09985
- WO-A-98/21327
- WO-A-99/67284
- WO-A1-99/58564
- US-A- 5 958 883
- QUIBELL, MARTIN ET AL: "Solid-phase assembly of backbone amide-protected peptide segments: an efficient and reliable strategy for the synthesis of small protein" JOURNAL OF THE CHEMICAL SOCIETY, PERKIN TRANSACTIONS 1: ORGANIC AND BIO-ORGANIC CHEMISTRY , CODEN: JCPRB4; ISSN: 0300-922X, vol. 11, 1996, pages 1227-1234, XP009067500
- BECK-SICKINGER A G ET AL: "Sulfonation of arginine residues as side reaction in Fmoc-peptide synthesis" INTERNATIONAL JOURNAL OF PEPTIDE AND PROTEIN RESEARCH 1991 DENMARK, vol. 38, no. 1, 1991, pages 25-31, XP000215705 ISSN: 0367-8377
- WAKIMASU M ET AL: "USE OF THE 4 METHOXY-2 6-DIMETHYL BENZENESULFONYL GROUP TO SYNTHESIZE DYNORPHIN 1-13 AND RELATED PEPTIDES" CHEMICAL AND PHARMACEUTICAL BULLETIN (TOKYO), vol. 29, no. 9, 1981, pages 2592-2597, XP001246926 ISSN: 0009-2363
- UKAI M ET AL: "Dynorphin A-(1-13) attenuates basal forebrain-lesion-induced amnesia in rats" BRAIN RESEARCH, vol. 625, no. 2, 22 October 1993 (1993-10-22), pages 355-356, XP002384245 ISSN: 0006-8993
- NASHABEH W ET AL: "Studies in capillary zone electrophoresis with a post-column multiple capillary device for fraction collection and stepwise increase in electroosmotic flow during analysis" ELECTROPHORESIS, WILEY-VCH VERLAG, vol. 14, no. 5/6, May 1993 (1993-05), pages 407-416, XP009005692 WEINHEIM, DE ISSN: 0173-0835
- PELSUE S ET AL: "Immunoreactivity between a monoclonal lupus autoantibody and the arginine/aspartic acid repeats within the U1-snRNP 70K autoantigen is conformationally restricted." JOURNAL OF PROTEIN CHEMISTRY. MAY 1994, vol. 13, no. 4, May 1994 (1994-05), pages 401-408, XP009005689 ISSN: 0277-8033
- BOWNESS P ET AL: "Identification of T cell receptor recognition residues for a viral peptide presented by HLA B27" EUROPEAN JOURNAL OF IMMUNOLOGY 1994 GERMANY, vol. 24, no. 10, 1994, pages 2357-2363, XP001031299 ISSN: 0014-2980
- YAMAMOTO K ET AL: "THE SURVIVAL OF RAT CEREBRAL CORTICAL NEURONS IN THE PRESENCE OF TROPHIC APP PEPTIDES" JOURNAL OF NEUROBIOLOGY, JOHN WILEY AND SONS., NEW YORK, NY, US, vol. 25, no. 5, May 1994 (1994-05), pages 585-594, XP001107074 ISSN: 0022-3034
- JIN L-W ET AL: "PEPTIDES CONTAINING THE RERMS SEQUENCE OF AMYLOID SS/A4 PROTEIN PRECURSOR BIND CELL SURFACE AND PROMOTE NEURITE EXTENSION" JOURNAL OF NEUROSCIENCE, NEW YORK, NY, US, vol. 14, no. 9, 1994, pages 5461-5470, XP000920917 ISSN: 0270-6474
- MULTHAUP G ET AL: "Characterization of the high affinity heparin binding site of the Alzheimer's disease beta A4 amyloid precursor protein (APP) and its enhancement by zinc(II)." JOURNAL OF MOLECULAR RECOGNITION, HEYDEN & SON LTD., vol. 8, no. 4, July 1995 (1995-07), pages 247-257, XP009005720 LONDON, GB ISSN: 0952-3499
- MILEUSNIC R ET AL: "The role of APP in memory formation" EUROPEAN JOURNAL OF NEUROSCIENCE, vol. 12, no. Supplement 11, 24 June 2000 (2000-06-24), page 314, XP009067508 & MEETING OF THE FEDERATION OF EUROPEAN NEUROSCIENCE SOCIETIES; BRIGHTON, UK; JUNE 24-28, 2000 ISSN: 0953-816X
- MILEUSNIC R ET AL: "The peptide sequence Arg-Glu-Arg, present in the amyloid precursor protein, protects against memory loss caused by Abeta and acts as a cognitive enhancer" EUROPEAN JOURNAL OF NEUROSCIENCE, vol. 19, no. 7, April 2004 (2004-04), pages 1933-1938, XP002383860 ISSN: 0953-816X
- MILEUSNIC R ET AL: "Amyloid precursor protein: from synaptic plasticity to Alzheimer's disease." ANNALS OF THE NEW YORK ACADEMY OF SCIENCES, vol. 1048, June 2005 (2005-06), pages 149-165, XP002384155 ISSN: 0077-8923
- RIST BEATE ET AL: "The bioactive conformation of neuropeptide Y analogues at the human Y-2-receptor." EUROPEAN JOURNAL OF BIOCHEMISTRY, vol. 247, no. 3, 1997, pages 1019-1028, XP001135256 ISSN: 0014-2956
- CURTAIN, CYRIL C. ET AL: "Fusogenic activity of amino-terminal region of HIV type 1 Nef protein" AIDS RESEARCH AND HUMAN RETROVIRUSES (1994), 10(10), 1231-40, October 1994 (1994-10), XP009005690
- NINOMIYA, HARUAKI ET AL: "Amino acid sequence RERMS represents the active domain of amyloid.beta./A4 protein precursor that promotes fibroblast growth" JOURNAL OF CELL BIOLOGY (1993), 121(4), 879-86, 1993, XP009005688 cited in the application
- JAMES, J. A. ET AL: "Basic amino acids predominate in the sequential autoantigenic determinant of the small nuclear 70K ribonucleoprotein" SCANDINAVIAN JOURNAL OF IMMUNOLOGY (1994), 39(6), 557-66, 1994, XP009005691
- MERRIFIELD B: "SOLID PHASE SYNTHESIS" SCIENCE, AMERICAN ASSOCIATION FOR THE ADVANCEMENT OF SCIENCE, US, vol. 232, 18 April 1986 (1986-04-18), pages 341-347, XP000985292 ISSN: 0036-8075

### SEQUENCE LISTING

<110> The Open university
<110> Rose, Steven Peter Russell
<110> Mileusnic, Radmila
<120> Polypeptides, derivatives and uses thereof
<130> RA/P301588WO
<140> PCT/GB02/xxxxx
   <141> 2002-04-17
<150> GB 0109558.7
<151> 2001-04-18
<150> GB 0120084.9
   <151> 2001-08-17
<1.50> US 09/998491
   <151> 2001-11-30
<150> GB 0207387.2
   <151> 2002-03-28
<160> 22
<170> Patentin ver. 2.1
<210> 1
   <211> 695
   <212> PRT
   <213> Homo sapiens
<400> 1
   Met Leu Pro Gly Leu Ala Leu Leu Leu Leu Ala Ala Trp Thr Ala Arg
   1               5                   10                  15
   Ala Leu Glu Val Pro Thr Asp Gly Asn Ala Gly Leu Leu Ala Glu Pro
               20                  25                  30
   Gln Ile Ala Met Phe Cys Gly Arg Leu Asn Met His Met Asn Val Gln
           35                  40                  45
   Asn Gly Lys Trp Asp Ser Asp Pro Ser Gly Thr Lys Thr Cys Ile Asp
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
   165 170 175
   Asp Lys Phe Arg Gly Val Glu Phe Val Cys Cys Pro Leu Ala Glu Glu
   180 185 190
   Ser Asp Asn Val Asp Ser Ala Asp Ala Glu Glu Asp Asp Ser Asp Val
   195 200 205
   Trp Trp Gly Gly Ala Asp Thr Asp Tyr Ala Asp Gly Ser Glu Asp Lys
   210 215 220
   Val Val Glu Val Ala Glu Glu Glu Glu Val Ala Glu Val Glu Glu Glu
   225 230 235 240
   Glu Ala Asp Asp Asp Glu Asp Asp Glu Asp Gly Asp Glu Val Glu Glu
   245 250 255
   Glu Ala Glu Glu Pro Tyr Glu Glu Ala Thr Glu Arg Thr Thr Ser Ile
   260 265 270
   Ala Thr Thr Thr Thr Thr Thr Thr Glu Ser Val Glu Glu Val Val Arg
   275 280 285
   Val Pro Thr Thr Ala Ala Ser Thr Pro Asp Ala Val Asp Lys Tyr Leu
   290 295 300
   Glu Thr Pro Gly Asp Glu Asn Glu His Ala His Phe Gln Lys Ala Lys
   305 310 315 320
   Glu Arg Leu Glu Ala Lys His Arg Glu Arg Met Ser Gln Val Met Arg
   325 330 335
   Glu Trp Glu Glu Ala Glu Arg Gln Ala Lys Asn Leu Pro Lys Ala Asp
   340 345 350
   Lys Lys Ala Val Ile Gln His Phe Gln Glu Lys Val Glu Ser Leu Glu
   355 360 365
   Gln Glu Ala Ala Asn Glu Arg Gln Gln Leu Val Glu Thr His Met Ala
   370 375 380
   Arg Val Glu Ala Met Leu Asn Asp Arg Arg Arg Leu Ala Leu Glu Asn
   385 390 395 400
   Tyr Ile Thr Ala Leu Gln Ala Val Pro Pro Arg Pro Arg His Val Phe
   405 410 415
   Asn Met Leu Lys Lys Tyr Val Arg Ala Glu Gln Lys Asp Arg Gln His
   420 425 430
   Thr Leu Lys His Phe Glu His Val Arg Met Val Asp Pro Lys Lys Ala
   435 440 445
   Ala Gln Ile Arg Ser Gln Val Met Thr His Leu Arg Val Ile Tyr Glu
   450 455 460
   Arg Met Asn Gln Ser Leu Ser Leu Leu Tyr Asn Val Pro Ala Val Ala
   465 470 475 480
   Glu Glu Ile Gln Asp Glu Val Asp Glu Leu Leu Gln Lys Glu Gln Asn
   485 490 495
   Tyr Ser Asp Asp Val Leu Ala Asn Met Ile Ser Glu Pro Arg Ile Ser
   500 505 510
   Tyr Gly Asn Asp Ala Leu Met Pro Ser Leu Thr Glu Thr Lys Thr Thr
   515 520 525
   Val Glu Leu Leu Pro Val Asn Gly Glu Phe Ser Leu Asp Asp Leu Gln
   530 535 540
   Pro Trp His Ser Phe Gly Ala Asp Ser Val Pro Ala Asn Thr Glu Asn
   545 550 555 560
   Glu Val Glu Pro Val Asp Ala Arg Pro Ala Ala Asp Arg Gly Leu Thr
   565 570 575
   Thr Arg Pro Gly Ser Gly Leu Thr Asn Ile Lys Thr Glu Glu Ile Ser
   580 585 590
   Glu Val Lys Met Asp Ala Glu Phe Arg His Asp Ser Gly Tyr Glu Val
   595 600 605
   His His Gln Lys Leu Val Phe Phe Ala Glu Asp Val Gly Ser Asn Lys
   610 615 620
   Gly Ala Ile Ile Gly Leu Met Val Gly Gly Val Val Ile Ala Thr Val
   625 630 635 640
   Ile Val Ile Thr Leu Val Met Leu Lys Lys Lys Gln Tyr Thr Ser Ile
   645 650 655
   His His Gly Val Val Glu Val Asp Ala Ala Val Thr Pro Glu Glu Arg
   660 665 670
   His Leu Ser Lys Met Gln Gln Asn Gly Tyr Glu Asn Pro Thr Tyr Lys
   675 680 685
   Phe Phe Glu Gln Met Gln Asn
   690 695
<210> 2
   <211> 534
   <212> PRT
   <213> Gallus gallus
<400> 2
   Gly Met Asn Leu His Asp Tyr Gly Met Leu Leu Pro Cys Gly Ile Asp
   1               5                   10                  15
   Lys Phe Arg Gly Val Glu Phe Val Cys Cys Pro Leu Ala Glu Glu Ser
               20                  25                  30
   Asp Asn Leu Asp Ser Ala Asp Ala Glu Asp Asp Asp Ser Asp Val Trp
           35                  40                  45
   Trp Gly Gly Ala Asp Ala Asp Tyr Ala Asp Gly Ser Asp Asp Lys Val
       50                  55                  60
   Val Glu Glu Gln Pro Glu Glu Asp Glu Glu Leu Thr Val Val Glu Asp
   65                  70                  75                  80
   Glu Asp Ala Asp Asp Asp Asp Asp Asp Asp Gly Asp Glu Ile Glu Glu
                   85                  90                  95
   Thr Glu Glu Glu Tyr Glu Glu Ala Thr Glu Arg Thr Thr Ser Ile Ala
               100                 105                 110
   Thr Thr Thr Thr Thr Thr Thr Glu Ser Val Glu Glu Val Val Arg Val
           115                 120                 125
   Pro Thr Thr Ala Ala Ser Thr Pro Asp Ala Val Asp Lys Tyr Leu Glu
       130                 135                 140
   Thr Pro Gly Asp Glu Asn Glu His Ala His Phe Gln Lys Ala Lys Glu
   145                 150                 155                 160
   Arg Leu Glu Ala Lys His Arg Glu Arg Met Ser Gln Val Met Arg Glu
   165 170 175
   Trp Glu Glu Ala Glu Arg Gln Ala Lys Asn Leu Pro Lys Ala Asp Lys
   180 185 190
   Lys Ala Val Ile Gln His Phe Gln Glu Lys Val Glu Ser Leu Glu Gln
   195 200 205
   Glu Ala Ala Asn Glu Arg Gln Gln Leu Val Glu Thr His Met Ala Arg
   210 215 220
   Val Glu Ala Met Leu Asn Asp Arg Arg Arg Ile Ala Leu Glu Asn Tyr
   225 230 235 240
   Ile Thr Ala Leu Gln Thr Val Pro Pro Arg Pro Arg His Val Phe Asn
   245 250 255
   Met Leu Lys Lys Tyr Val Arg Ala Glu Gln Lys Asp Arg Gln His Thr
   260 265 270
   Leu Lys His Phe Glu His Val Arg Met Val Asp Pro Lys Lys Ala Ala
   275 280 285
   Gln Ile Arg Ser Gln Val Met Thr His Leu Arg Val Ile Tyr Glu Arg
   290 295 300
   Met Asn Gln Ser Leu Ser Phe Leu Tyr Asn Val Pro Ala Val Ala Glu
   305 310 315 320
   Glu Ile Gln Asp Glu Val Asp Glu Leu Leu Gln Lys Glu Gln Asn Tyr
   325 330 335
   Ser Asp Asp Val Leu Ala Asn Met Ile Ser Glu Pro Arg Ile Ser Tyr
   340 345 350
   Gly Asn Asp Ala Leu Met Pro Ser Leu Thr Glu Thr Lys Thr Thr Val
   355 360 365
   Glu Leu Leu Pro Val Asp Gly Glu Phe Ser Leu Asp Asp Leu Gln Pro
   370 375 380
   Trp His Pro Phe Gly Val Asp Ser Val Pro Ala Asn Thr Glu Asn Glu
   385 390 395 400
   Val Glu Pro Val Asp Ala Arg Pro Ala Ala Asp Arg Gly Leu Thr Thr
   405 410 415
   Arg Pro Gly Ser Gly Leu Thr Asn Val Lys Thr Glu Glu Val Ser Glu
   420 425 430
   Val Lys Met Asp Ala Glu Phe Arg His Asp Ser Gly Tyr Glu Val His
   435 440 445
   His Gln Lys Leu Val Phe Phe Ala Glu Asp Val Gly Ser Asn Lys Gly
   450 455 460
   Ala Ile Ile Gly Leu Met Val Gly Gly Val Val Ile Ala Thr Val Ile
   465 470 475 480
   Val Ile Thr Leu Val Met Leu Lys Lys Lys Gln Tyr Thr Ser Ile His
   485 490 495
   His Gly Val Val Glu Val Asp Ala Ala Val Thr Pro Glu Glu Arg His
   500 505 510
   Leu Ser Lys Met Gln Gln Asn Gly Tyr Glu Asn Pro Thr Tyr Lys Phe
   515 520 525
   Phe Glu Gln Met Gln Asn
       530
<210> 3
   <211> 5
   <212> PRT
   <213> Artificial sequence
<220>
   <223> Description of Artificial Sequence: 5-mer polypeptide
<400> 3
   Arg Glu Arg Met Ser
    1               5
<210> 4
   <211> 5
   <212> PRT
   <213> Artificial Sequence
<220>
   <223> Description of Artificial Sequence: 5-mer polypeptide
<400> 4
   Ser Met Arg Glu Arg
    1               5
<210> 5
   <211> 5
   <212> PRT
   <213> Artificial Sequence
<220>
   <223> Description of Artificial Sequence: 5-mer polypeptide
<400> 5
   Arg Ser Ala Glu Arg
    1               5
<210> 6
   <211> 17
   <212> PRT
   <213> Artificial Sequence
<220>
   <223> Description of Artificial sequence: 17-mer polypeptide
<400> 6 <211> 17
   <212> PRT
   <213> Artificial Sequence
<220>
   <223> Description of Artificial Sequence: 17-mer polypeptide
<400> 7
<210> 8
   <211> 17
   <212> PRT
   <213> Artificial Sequence
<220>
   <223> Description of Artificial Sequence: 17-mer polypeptide
<400> 8
   Val His His Gln Lys Leu Val Phe Phe Ala Glu Asp Val Gly Ser Asn
   1 5 10 15
   Lys
<210> 9
   <211> 3
   <212> PRT
   <213> Artificial Sequence
<220> <223> Description of Artificial Sequence: 3-mer polypeptide
<400> 9
   Arg Glu Arg
   1
<210> 10
   <211> 4
   <212> PRT
   <213> Artificial Sequence
<220>
   <223> Description of Artificial sequence: 4-mer polypeptide
<400> 10
   Arg Glu Arg Met
   1
<210> 11
   <211> 4
   <212> PRT
   <213> Artificial Sequence
<220>
   <223> Description of Artificial Sequence: 4-mer polypeptide
<400> 11
   Met Arg Glu Arg
   1
<210> 12
   <211> 16
   <212> PRT
   <213> Artificial Sequence
<220>
   <223> Description of Artificial Sequence: 16-mer polypeptide
<400> 12
   Gln Lys Ala Lys Glu Arg Leu Glu Ala Lys His Arg Glu Arg Met Ser
   1 5 10 15
<210> 13
   <211> 15
   <212> PRT
   <213> Artificial Sequence
<220>
   <223> Description of Artificial Sequence: 15-mer polypeptide
<400> 13
   Glu Arg Leu Glu Ala Lys His Arg Glu Arg Met Ser Gln Val Met
   1 5 10 15
<210> 14
   <211> 14
   <212> PRT
   <213> Artificial Sequence
<220>
   <223> Description of Artificial Sequence: 14-mer polypeptide
<400> 14
   Ala Lys Glu Arg Leu Glu Ala Lys His Arg Glu Arg Met Ser
   1 5 10
<210> 15
   <211> 12
   <212> PRT
   <213> Artificial Sequence
<220> <223> Description of Artificial Sequence: 12-mer polypeptide
<400> 15
   Glu Arg Leu Glu Ala Lys His Arg Glu Arg Met Ser
   1 5 10
<210> 16
   <211> 11
   <212> PRT
   <213> Artificial Sequence
<220>
   <223> Description of artificial Sequence: 11-mer polypeptide
<400> 16
   Glu Arg Leu Glu Ala Lys His Arg Glu Arg Met
   1 5 10
<210> 17
   <211> 11
   <212> PRT
   <213> Artificial Sequence
<220> <223> Description of Artificial Sequence: 11-mer polypeptide
<400> 16
<210> 18
   <211> 10
   <212> PRT
   <213> Artificial Sequence
<220>
   <223> Description of Artificial Sequence: 10-mer polypeptide
<400> 18
   Glu Arg Leu Glu Ala Lys His Arg Glu Arg
   1 5 10
<210> 19
   <211> 6
   <212> PRT
   <213> Artificial Sequence
<220>
   <223> Description of Artificial Sequence: 6-mer polypeptide
<400> 19
   His Arg Glu Arg Met Ser
   1 5
<210> 20
   <211> 22
   <212> PRT
   <213> Artificial Sequence
<220>
   <223> Description of Artificial Sequence: 22-mer polypeptide
<400> 20
   Phe Gln Lys Ala Lys Glu Arg Leu Glu Ala Lys His Arg Glu Arg Met
   1 5 10 15
   Ser Gln Val Met Arg Glu
   20
<210> 21
   <211> 16
   <212> DNA
   <213> Artificial sequence
<220>
   <223> Description of Artificial Sequence: end-protected 16-mer oligodeoxynucleotide modified at positions 2 & 13 to prevent internal looping
<400> 21
   cccgaggact gagcca 16
<210> 22
   <211> 40
   <212> PRT
   <213> Artificial sequence
<220>
   <223> Description of Artificial Sequence: 40-mer polypeptide
<400> 22
   Thr Pro Asp Ala Val Asp Lys Tyr Leu Glu Thr Pro Gly Asp Glu Asn
    1               5                  10                  15
   Glu His Ala His Phe Gln Lys Ala Lys Glu Arg Leu Glu Ala Lys His
               20                  25                  30
   Arg Glu Arg Met Ser Gln Val Met
            35                  40

## Description

### FIELD OF THE INVENTION

The present invention relates to polypeptide compounds, to compounds derived therefrom and to the use of such compounds in medicine. Compounds according to the invention are believed to be potentially useful as cognitive enhancers and in the treatment of neurodegenerative diseases such as Alzheimer's disease.

### BACKGROUND TO THE INVENTION

Alzheimer's disease is a degenerative brain disease which is characterised by progressive loss of memory and subsequently most other cognitive functions in an irreversible decline over a period of years. It represents a substantial health problem, particularly in an ageing population.

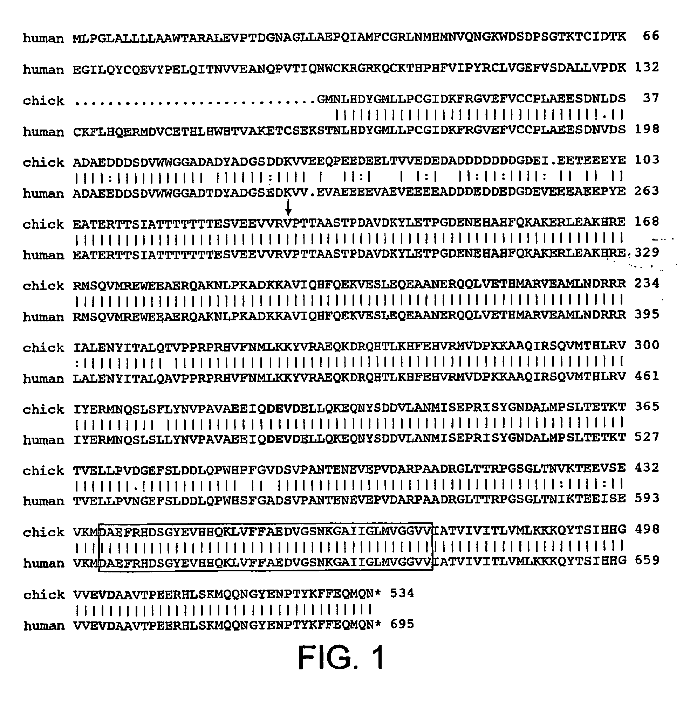

The amyloid precursor protein ("APP") is a multifunctional transmembrane protein and is known to have important functions in normal brain tissue. The human form of APP is known to consist of 695 amino acid residues (SEQ ID No: 1) in a sequence which is also known (see Kang et al, Nature 325, 733-736 (1987). The chick form of APP is known to consist of 534 amino acid residues (SEQ ID No: 2) and to resemble the human form closely, being approximately 95% homologous therewith (see the paper by Kang et al just mentioned and Barnes et al, J Neurosci, 18 (15) 5869-5880 (1998). The amino acid sequences of the human and chick forms of APP are reproduced in figure 1 of the drawings of this specification.

Two effects which have been noted to take place in the brain of a person suffering from Alzheimer's disease are the build up outside the nerve cells of the brain of tangled masses of protein and the build up inside the brain cells of a different protein. The extracellular proteins are known to be aggregates of polypeptides having amino acid sequences corresponding to portions of the extracellular part of APP. The tangled masses of these proteins are known as amyloid plaques. The intracellular proteins are known as tau proteins. It is however not known whether either or both of the extracellular accumulation of amyloid plaques and the intracellular accumulation of tau proteins are the causes or the symptoms of Alzheimer's and related neurodegenerative diseases of the Alzheimer type.

The amino acid sequence of the β-amyloid polypeptide fragment (1-42) is identical in the human and chick forms of APP and consists of amino acid residues 597 to 638 in the human form and residues 436 to 477 in the chick form, (see the papers by Kang et al and Barnes et al referred to hereinbefore).

### DEFINITIONS

The following expressions are used in this specification and have the following meanings, except where the context indicates otherwise:

| | |
|---|---|
| **APP** | means "amyloid precursor protein"; |
| **human APP** | means the human form of APP; |
| **chick APP** | means the chick form of APP; |
| **RERMS** | means the pentapeptide Arg-Glu-Arg-Met-Ser (SEQ ID No: 3); |
| **APP 328-332** | also means the pentapeptide Arg- |
| | Glu-Arg-Met-Ser (SEQ ID No: 3) which corresponds to amino acid residues 328 to 332 of human APP; |
| **SMRER** | means the pentapeptide Ser-Met-Arg-Glu-Arg (SEQ ID No: 4); |
| **APP 332-328** | means the same as SMRER; |
| **A**β **domain** | means the domain of APP which forms β-amyloid plaques; |
| β**-amyloid 12-28** | means the sequence of amino acid residues which constitute part of the Aβ domain of human APP, the sequence being Val-His-His-Gln-Lys-Leu-Val-Phe-Phe-Ala-Glu-Asp-Val-Gly-Ser-Asn-Lys( SEQ ID No: 8) which corresponds to amino acid residues 608 to 624 of human APP and amino acids 447 to 463 of chick APP; |
| **RSAER** | means the pentapeptide Arg-Ser-Ala-Glu-Arg (SEQ ID No: 5); |
| **APP 319-335** | means the polypeptide Ala-Lys-Glu-Arg-Leu-Glu-Ala-Lys-His-Arg-Glu-Arg-Met-Ser-Gln-Val-Met (AKERLEAKHRERMSQVM)(SEQ ID No: 6) ; |
| **RER** | means the tripeptide Arg-Glu-Arg (SEQ ID No: 9); |
| **APP 328-330** | means the same as RER; |
| **Ac-RER** | means RER in which one of the hydrogen atoms of the -NH₂ group at the N-terminus has been replaced by an acetyl group; the structural formula of Ac-RER is given hereinbelow; |
| **RERM** | means the tetrapeptide Arg-Glu- |
| | Arg-Met (SEQ ID No: 10); |
| **APP 328-331** | means the same as RERM; |
| **MRER** | means the tetrapeptide Met-Arg-Glu-Arg (SEQ ID No: 11); |
| **APP 331-328** | means the same as MRER; |
| **amino acid** | as used herein is meant to include both natural and synthetic amino acids, and both D and L amino acids; |
| **standard amino acid** | means any of the twenty standard L-amino acids commonly found in naturally occurring peptides; |
| **nonstandard amino acid** | means any amino acid, other than the standard amino acids, regardless of whether it is prepared synthetically or derived from a natural source; as used herein, "synthetic amino acid" also encompasses chemically modified amino acids, including but not limited to salts, amino acid derivatives (such as amides), and substitutions. Amino acids contained within the peptides of the present invention, and particularly at the carboxy- or amino-terminus, can be modified by methylation, amidation, acetylation or substitution with other chemical groups which can change the peptide's circulating half life without adversely affecting their activity; additionally, a disulfide linkage may be present |
| | or absent in the peptides of the invention; |
| **derivative** | includes any purposefully generated peptide which in its entirety, or in part, has a substantially similar amino acid sequence to the present compounds; derivatives of the present compounds may be characterized by single or multiple amino acid substitutions, deletions, additions, or replacements; these derivatives may include (a) derivatives in which one or more amino acid residues of the present compounds are substituted with conservative or non-conservative amino acids; (b) derivatives in which one or more amino acids are added to the present compounds; (c) derivatives in which one or more of the amino acids of the present compounds include a substituent group; (d) derivatives in which the present compounds or a portion thereof is fused to another peptide (e.g., serum albumin or protein transduction domain); (e) derivatives in which one or more nonstandard amino acid residues (i.e., those other than the 20 standard L-amino acids found in naturally |
| | occurring proteins) are incorporated or substituted into the present compounds sequence; and (f) derivatives in which one or more nonamino acid linking groups are incorporated into or replace a portion of the present compounds; |
| **APP 296-335, APP 317-332, APP 321-335, APP 319-332, APP 321-332, APP 321-331, APP 325-335, APP 321-330, APP 327-332 and APP 316-337** | have the meanings given in tables 1 and 2 hereinafter. |

Throughout this specification and its claims amino acid sequences are written using the standard one-letter or three-letter abbreviations. All sequences are written from left to right in the direction from the N-terminus to the C-terminus.
The following term is defined as follows:

| | |
|---|---|
| **reverse order sequence** | as used herein, the reverse order sequence of a given sequence is a sequence in which the order of amino acid residues is reversed compared with the given sequence when reading in the direction from the N-terminus to the C-terminus and vice *versa*; thus, for example, SMRER is the reverse order sequence of RERMS, each being read as stated above from left to right in the N-terminal to C-terminal direction; further, MVQSMRERHKAELREKA (SEQ ID No: 7) (also referred to herein as APP 335-319) is the reverse order sequence of APP 319-335 defined above. |

WO-A-94/09808 (The Regents of The University of California, inventor T Saitoh) and T Saitoh and various coauthors in J. Neuroscience 14 5461-5470 (1994), J. Neurobiol. 25, 585-594 (1994) and J. Cell Biol. 121, 879-886 (1993) disclose certain polypeptides corresponding to parts of human APP. These polypeptides consist of the following:

**Table 1**

| no. of amino acid residues | corresponding part of human APP | sequence | SEQ ID NO. |
|---|---|---|---|
| 40 | APP 296-335 | | 22 |
| 17 | APP 319-335 | AKERLEAKHRERMSQVM | 6 |
| | | | |
| 16 | APP 317-332 | QKAKERLEAKHRERMS | 12 |
| | | | |
| 15 | APP 321-335 | ERLEAKHRERMSQVM | 13 |
| | | | |
| 14 | APP 319-332 | AKERLEAKHRERMS | 14 |
| | | | |
| 12 | APP 321-332 | ERLEAKHRERMS | 15 |
| | | | |
| 11 | APP 321-331 | ERLEAKHRERM | 16 |
| | | | |
| 11 | APP 325-335 | AKHRERMSQVM | 17 |
| | | | |
| 10 | APP 321-330 | ERLEAKHRER | 18 |
| | | | |
| 6 | APP 327-332 | HRERMS | 19 |
| | | | |
| 5 | APP 328-332 | RERMS, | 3 |
| | | [Arg-Glu-Arg-Met-Ser] | |
| 4 | APP 328-331 | RERM | 10 |
| | | [Arg-Glu-Arg-Met] | |
| 3 | APP 328-330 | RER | 9 |
| | | [Arg-Glu-Arg] | |

The same publications also refer to a 17-mer polypeptide which is the reverse-order sequence of APP 319-335 (as defined above). The reverse order sequence is also identified herein as APP 335-319.

G Multhaup et al in J. Mol Recognition 8, 247-257 (1995) disclose the following polypeptide which also corresponds to part of human APP:

**Table 2**

| No. of amino acid residues | corresponding part of human APP | Sequence | SEQ ID NO. |
|---|---|---|---|
| 22 | APP 316-337 | FQKAKERLEAKHRERMSQVMRE | 20 |
| | | | |

MILEUSNIC R et al., EUROPEAN JOURNAL OF NEUROSCIENCE, vol. 12, no. Supplement 11, page 314, (24 June 2000) discloses that the injection of RERMS or SMRER 30 min before memory training (one trial passive avoidance task: pre-training day-old chicks) reverses the APP antisense - induced amnesia in said chicks (Abstract).

WO-A-97/00063 discloses a peptide comprising the sequence RER at its C-terminus (page 61, example XXI), which is stated (page 61, lines 7-9) to be a potentially superior blocking agent for increasing the available amount of CRF and/or Ucn, which latter is stated (page 61, lines 24-27) to find application in modifying the mood, learning, memory and behaviour of normally and mentally disordered (Alzheimer's disease: page 64, lines 22-24) individuals (page 61, lines 24-27, claim 36). A substituted amide (methylamide, ethylamide) may be incorporated at the C-terminus (page 67, lines 7-10).

### SUMMARY OF THE INVENTION

The present invention provides a pharmaceutical composition comprising: (a) a compound having the formula: 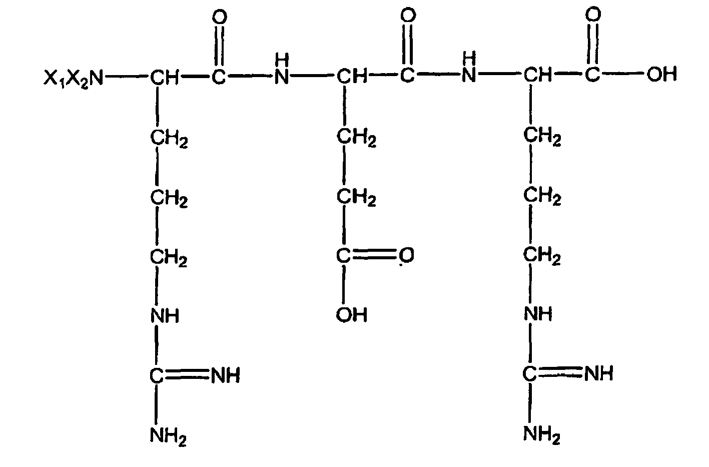 in which X₁ is an acyl group represented by the formula: 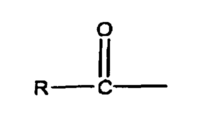 X₂ is H or an acyl group represented by the formula: 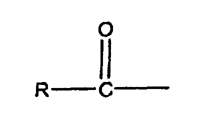 and R represents a straight- or branched-chain alkyl group, an alkyl-substituted or unsubstituted cycloalkyl group, a straight or branched-chain aralkyl group or an alkyl-substituted
or unsubstituted aryl group, the groups R being the same or different when two are present, and (b) a pharmaceutically acceptable carrier.

The invention also provides a non-therapeutic, non-surgical method of producing a cognitive enhancement effect in an animal, comprising
providing an animal in which the effect is to be produced; and
administering to the animal an amount of a compound effective to produce the cognitive enhancement, the compound having the formula: 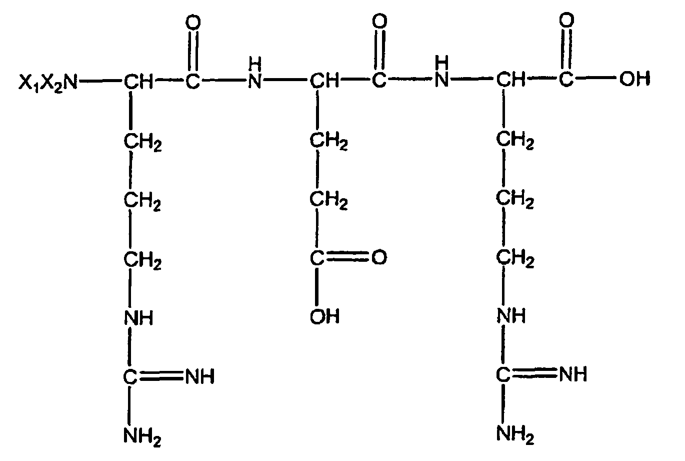 in which X₁ is an acyl group represented by the formula: 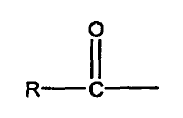 X₂ is H or an acyl group represented by the formula: 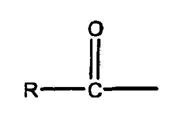 and R represents a straight- or branched-chain alkyl group, an alkyl-substituted or unsubstituted cycloalkyl group, a straight or branched-chain aralkyl group or an alkyl-substituted or unsubstituted aryl group, the groups R being the same or different when two are present.
Preferably, X₂ is H.
Advantageously, R represents a C₁ to C₆ straight- or branched-chain alkyl group.
More preferably, R is a methyl group.

Examples of straight- and branched-chain alkyl groups are methyl, ethyl, n-propyl, isopropyl, n-butyl, isobutyl, s-butyl, t-butyl, pentyl and hexyl groups. Examples of substituted or unsubstituted cycloalkyl groups are methylcyclohexyl and cyclohexyl groups. Examples of substituted or unsubstituted straight- or branched-chain aralkyl groups are a benzyl group. Examples of substituted or unsubstituted aryl groups are phenyl and tolyl groups.

Straight- or branched-chain alkyl groups are most preferred for R.

The compound of the invention may be physically linked to a further molecule or vehicle for pharmaceutical delivery of the compound.

The invention further provides a compound as defined above, for use in medicine and the use of such a compound in the preparation of medicaments for the treatment of neurodegenerative diseases, including Alzheimer's disease, and as a cognitive enhancer. The medicinal use is preferably the treatment of a neurodegenerative disease, more preferably the treatment of Alzheimer's disease.

### BRIEF DESCRIPTION OF THE DRAWINGS

The drawings of this specification consist of the following:
Figure 1 which shows the amino acid sequence of human APP and chick APP, as referred to hereinbefore;
Figure 2 shows the effect of β-amyloid 12-28 polypeptide on memory formation;
Figure 3 shows the effect of RERMS on β-amyloid 12-28 induced amnesia;
Figure 4 shows the effect of RERMS on anti-APP induced amnesia;
Figure 5 shows the effect of RERMS, SMRER and RSAER on APP-antisense induced amnesia;
Figure 6 shows the effect of APP 319-335 on APP-antisense induced amnesia;
Figure 7 shows the effect of RERMS on weak training;
Figure 8 shows the effect of APP 319-335 on weak training;
Figure 9 shows the effect of RER on weak training;
Figure 10 shows the effects of RER and Ac-RER on β-amyloid 12-28 induced amnesia;
Figure 11 shows the effect of Ac-RER on weak training;
Figure 12 shows the effects of differing doses of RER and Ac-RER; and
Figure 13 shows the effect of Ac-RER on β-amyloid 12-28 induced amnesia.

### DETAILED DESCRIPTION OF THE INVENTION

The invention will now be described further by way of example with reference to the following experimental procedures and results. The peptides RER, RERMS, SMRER, RSAER and APP 319-335 refrrred to fall outside the scope of the invention.

### Materials and methods

### Animals and training

Commercially obtained Ross Chunky eggs were incubated and hatched in brooders and held until 16 ±6 hours old. Chicks were placed in pairs in small aluminium pens. Following an equilibration period of an hour, the chicks were pretrained and trained essentially as described by Lossner and Rose (J. Neurochem. 41, 1357-1363 (1983). Pretraining involved three 10 s presentations of a small (2 mm diameter) white bead, at approximately 5 minute intervals. Chicks, which failed to peck the bead at least twice in three presentations (less than 5%), were not used subsequently, but remained in their pens for the duration of the experiment. Two training techniques were used: "strong" and "weak" training. In both, 5 to 10 minutes after the last pre-training trial, chicks were trained by a 10 s presentation of a 4 mm diameter chrome bead, which had been dipped in the bitter-tasting methylanthranilate. Control chicks pecked at a water-coated or dry bead. In the "strong" version of the task, 100% methylanthranilate was used. In the "weak" version, 10% methylanthranilate was used. Chicks spontaneously pecked at the training or control beads within 20 s. Chicks that peck at the bitter bead evinced a disgust reaction and would not normally peck at a similar, but dry bead for some hours subsequently. At various times following training chicks were tested, by offering them a dry 4 mm diameter chrome bead, followed 10 minutes later by a small (2 mm diameter) white bead, each for 20 to 30 s. Animals were tested by an experimenter blind as to which treatment each chick had received. Chicks are considered to remember the task if they avoid the chrome bead at test but peck at the white bead (discriminate), and to have forgotten it if they peck at both beads. Recall is calculated as a percent avoidance score(percentage of chicks which avoid the chrome bead) and as a discrimination score (percentage of chicks which avoid the chrome but peck at the white bead). The use of the discrimination score ensures that chicks can indeed see and peck accurately at the bead; and hence that the avoidance of the chrome bead is not due to non-specific factors such as lack of visuo-motor coordination, motivation, attention, arousal, etc. but is a positive act, demonstrating memory for the distasteful stimulus. Each chick was trained and tested only once and differences between groups tested for statistical significance by g-test described by Sokal and Rohlf (Biometry: the Principles and Practice of Statistics in Biological Research (2nd edition), W H Freeman, New York (1981)), .

The validity of this particular training task used to assess memory formation is extensively discussed by Andrew (Neural and Behavioural Plasticity: the Use of the Domestic Chick as a Model, Oxford University Press, Oxford, UK (1991).

Chicks trained on the strong version of the task were found to recall the avoidance for at least 48 hours, and more than 80% were found normally to avoid and discriminate on test at 24 hours. Therefore if agents that are amnesic - that is, cause the chick not to remember - are administered, chicks will demonstrate forgetting by pecking rather than avoiding the chrome bead on test. By contrast, chicks were found normally to remember the "weak" version of the task for only a few hours - some 6 to 8 hours in all; retention at 24 hours was normally reduced to some 20 to 30%. Thus the learning experience is not committed to long-term memory. Agents that are memory enhancers can thus be tested. A memory enhancing agent, administered to a chick trained on the weak learning task, produces an increase in retention - increased avoidance of the chrome bead - at 24 hours. That is, such memory enhancers help convert weak to strong learning by enabling the transition from shorter to longer-term memory.

### Peptide injections

Bilateral intracranial injections (2 µg/hemisphere) of either saline, or solutions in saline of different peptides (0.5 to 5 µg/hemisphere) homologous to different regions of the external domain of human APP were injected intracerebrally into a specific brain region, known to be required for memory formation (the intermediate hyperstriatum ventrale) at different time-points pre- or post-training using a 5µg Hamilton syringe fitted with a plastic sleeve to allow a penetration of 3 mm. After completion of the injection, the needle was kept in place for 5 s. Correct placement was ensured by using a specially designed headholder described by Davis et al (Physiol. Behav. 22, 177-184 (1979), and was routinely visually monitored postmortem. Peptides or other substances were administered at various times either before or after the training protocol. Chicks were tested at different time points post-training as described above. The general behaviour of the chicks following injections was observed to detect any potential non-specific or adverse reactions to the injections.

### Peptide Materials

The polypeptides administered were synthesised using a conventional peptide synthesiser in a manner which is well-known to those skilled in the art. The synthesised polypeptides were purified by use of RP-HPLC and purity further checked by mass spectrometry (MALDI-TOF), both techniques being well known to those skilled in the art. The polypeptides after synthesis were kept under argon in a lyophilised state, the argon preventing oxidation of cysteine, methionine and tryptophan in particular.

Polypeptide synthesis as just mentioned is carried out by MWG-Biotech UK Limited of Milton Keynes, UK.

RERMS is also available from Bachem Limited of St. Helens, Merseyside, UK.

Ac-RER can be synthesised by techniques well-known to those skilled in the art. It was obtained from Cambridge Research Biochemicals Limited of Billingham, Cleveland, UK.

For further details regarding synthetic methods for the preparation of peptides and peptide derivatives, reference is made to "Principles of Peptide Synthesis" by M. Bodanszky, 2nd Edition (Springer Laboratory, 1993), .

### Experimental results

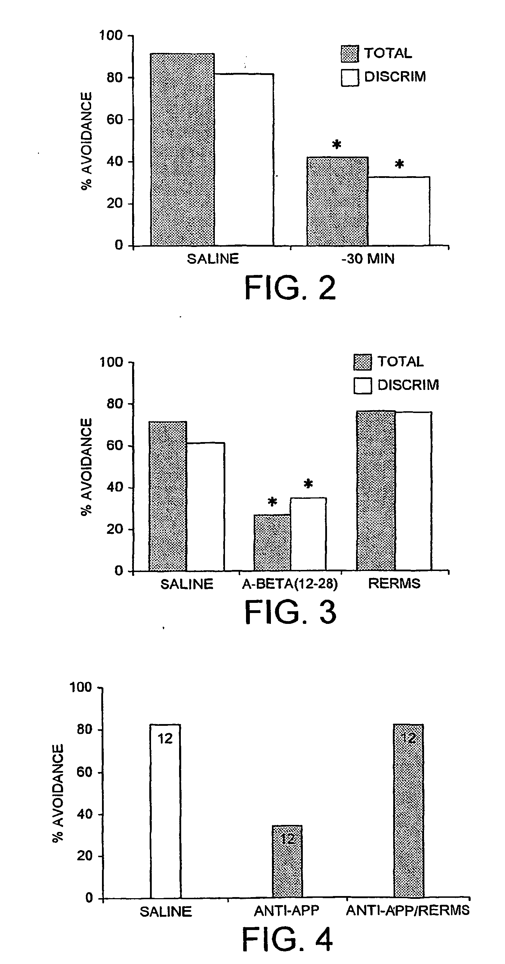

It is well known in many animal model systems for the study of memory that injection of β-amyloid and β-amyloid peptides, such as β-amyloid 12-28, results in a failure of animals to retain recently acquired memories. Figure 2 shows this result for a chick; injection of β-amyloid 12-28 into the brain 30 minutes prior to training chicks on the passive avoidance task results in amnesia in animals tested 30 minutes subsequently.

Figure 2 shows in the left-hand half the percent avoidance measured in terms of total avoidance and discrimination for a saline control and in the right-hand half the percent avoidance measured when β-amyloid 12-28 is injected as described above 30 minutes pretraining and memory is tested 30 minutes posttraining.

However, if amnesia is induced by injection of β-amyloid 12-28 30 minutes pretraining, and RERMS is injected 20 minutes pretraining, memory retention is restored. Figure 3 shows that in this case memory is normal at 24 hours post-training.

Figure 3 shows on the left the percent avoidance measured in terms of total avoidance and discrimination for a saline control, in the centre the corresponding results when β-amyloid 12-28 is injected 30 minutes pretraining and memory tested 24 hours posttraining, and on the right the results when the pretraining injection of β-amyloid 12-28 is followed 10 minutes later by RERMS and memory is again tested 24 hours posttraining.

It is thus shown that RERMS can prevent the memory loss produced by β-amyloid 12-28, a component of the amyloid plaques characteristic of Alzheimer's disease.

It is known that disrupting the normal function of APP by blocking its external domain with a specific monoclonal antibody (mb22C11) around the time of training, whilst without effect on the ability of chicks to learn the passive avoidance response, prevents the transition to long term memory. The monoclonal antibody mb22C11, available from Boehringer-Mannheim, specifically recognises an epitope consisting of part of the external domain of APP.

Figure 4 shows on the left the percent avoidance measured for chicks injected with a saline control, in the centre the percent avoidance measured when mb22C11 is injected (1-5 µg in 2 µl) intracerebrally as described hereinbefore for peptide injections 30 minutes pretraining and, on the right, the percent avoidance measured when RERMS is also injected 25 minutes after mb22C11 (5 minutes pretraining). In all cases, memory was tested 24 hours posttraining.

The results shown in figure 4 demonstrate that RERMS injected 5 minutes before training will prevent antibody induced memory loss and that the peptide RERMS can prevent anti-APP induced memory loss. Thus, RERMS can prevent the memory loss resulting from disrupting the normal function of

APP.

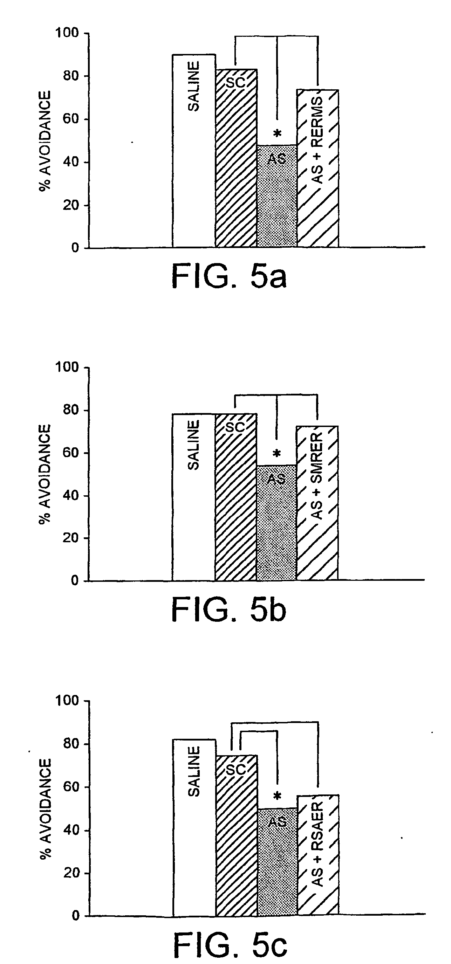

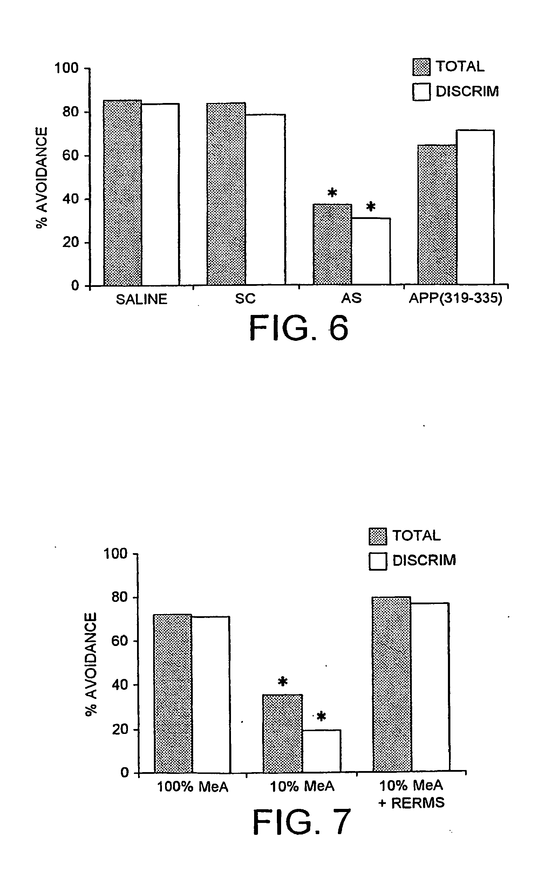

Figures 5 and 6 show the effect of inducing memory loss by injection of a 16-mer end-protected phosphodiester oligodeoxynucleotide designed to correspond to the transcription start sites 146 and AUG₁₇₈₆ of the APP mRNA, immediately upstream of a ribozyme binding site. The oligodeoxynucleotide, 5'-CCC GAG GAC TGA GCC A-3' (SEQ ID No: 21) was further modified on the 2nd and 13th nucleotides to prevent internal looping and is available from King's College Molecular Medicine Unit, London, UK. The oligodeoxynucleotide was used in scrambled (SC) and antisense (AS) forms and administered as described hereinbefore for peptide administration in an amount of 0.6 to 1.0 µg in 2 µl.

In a first experiment, chicks were injected with saline, RERMS, SMRER and RSAER in various combinations in the amounts stated hereinbefore.

The results are shown in figure 5 which shows the percent avoidances measured on the "strong" learning task described hereinbefore. Figure 5a shows the effect compared with a saline control of administration separately of SC oligodeoxynucleotide 12 hours pretraining and AS oligodeoxynucleotide 12 hours pretraining, together with the effect of administration of RERMS following the AS oligodeoxynucleotide 30 minutes pretraining.

Figure 5a shows that the SC oligodeoxynucleotide had no effect on memory but the AS compound had a significant effect of memory loss which was avoided to a substantial extent when RERMS was administered.

Figure 5b shows that similar results were obtained with the reverse-order pentapeptide SMRER.

Figure 5c shows that the effect obtained with RERMS and SMRER is absent with the pentapeptide RSAER.

In a second experiment, SC and AS oligodeoxynucleotides were administered 12 hours pretraining. A polypeptide (APP 319-335) corresponding to amino acid residues 319 to 335 of human APP was injected 30 minutes pretraining. Chicks were tested for memory according to the "strong" version of the test described hereinbefore 30 minutes posttraining.

Figure 6 shows successively from the left: the percent avoidance measured for a saline control; the percent avoidance measured when SC oligodeoxynucleotide was administered; the percent avoidance measured when AS oligodeoxynucleotide was administered; and the percent avoidance measured when APP 319-335 was administered 30 minutes pretraining following administration of AS oligodeoxynucleotide 12 hours pretraining. Each result is shown both in terms of total avoidance (left-hand column) and discrimination (right-hand column).

The results shown in figure 6 demonstrate that APP319-335 can prevent antisense induced memory loss.

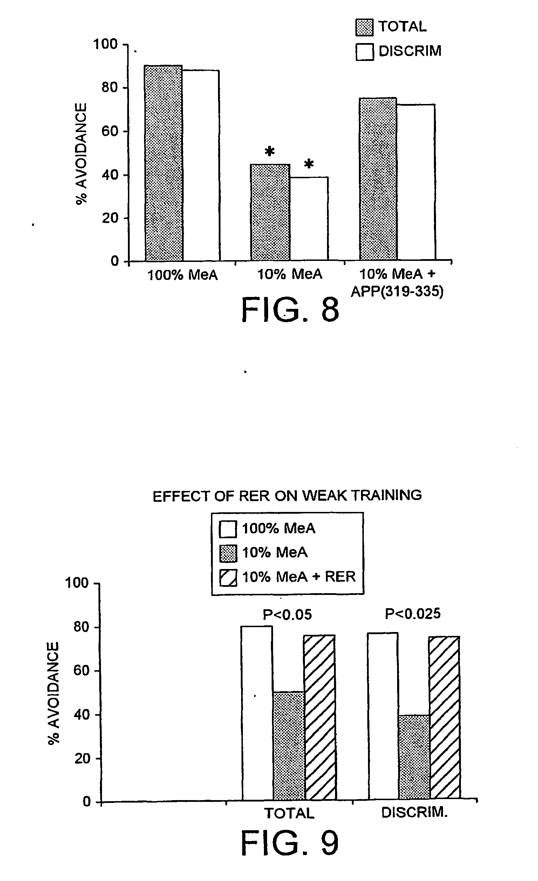

Figures 7 and 8 show the effects of RERMS and APP 319-335 on memory in chicks trained on the "weak" memory test described hereinbefore.

As stated hereinbefore, weakly trained chicks (trained on 10% methylanthranilate) retain memory for the avoidance for only some 6 hours, and thereafter forget. Figure 7 shows, on the left, the percent avoidance results (in terms of total avoidance and discrimination) for chicks trained on the "strong" version of the training, in the centre the corresponding results for "weak," training and, on the right, the effect of administration of RERMS following "weak" training. The chicks were tested for memory 24 hours posttraining; RERMS was administered in accordance with the procedure described hereinbefore 30 minutes pretraining.

Figure 8 shows the corresponding results obtained when the APP 319-335 polypeptide was used instead of RERMS.

Figures 7 and 8 show that RERMS and APP 319-335 if injected prior to training chicks on the weak task, enhance memory at 24 hours. They thus function as cognitive enhancers (nootropic agents). Thus, RERMS and APP 319-335 both enhance normal memory in weakly trained animals.

Figure 9 shows the effect of RER on memory in chicks trained on the "weak" memory test described hereinbefore.

As stated hereinbefore, weakly trained chicks (trained on 10% methylanthranilate) retain memory for the avoidance for only some 6 hours, and thereafter forget.

Figure 9 shows, in the three columns on the left, on the left the percent avoidance results (in terms of total avoidance) for chicks trained on the "strong" version of the training, in the centre the corresponding results for "weak" training and, on the right, the effect of administration of RER following "weak" training. The chicks were tested for memory 24 hours posttraining; RER was administered in accordance with the procedure described hereinbefore 30 minutes pretraining.

Figure 9 shows, on the right, the corresponding data in terms of discrimination.

Figure 9 shows that RER, if injected prior to training chicks on the weak task, enhances memory at 24 hours. It thus functions as a cognitive enhancer (nootropic agent).
Thus, RER enhances normal memory in weakly trained animals.

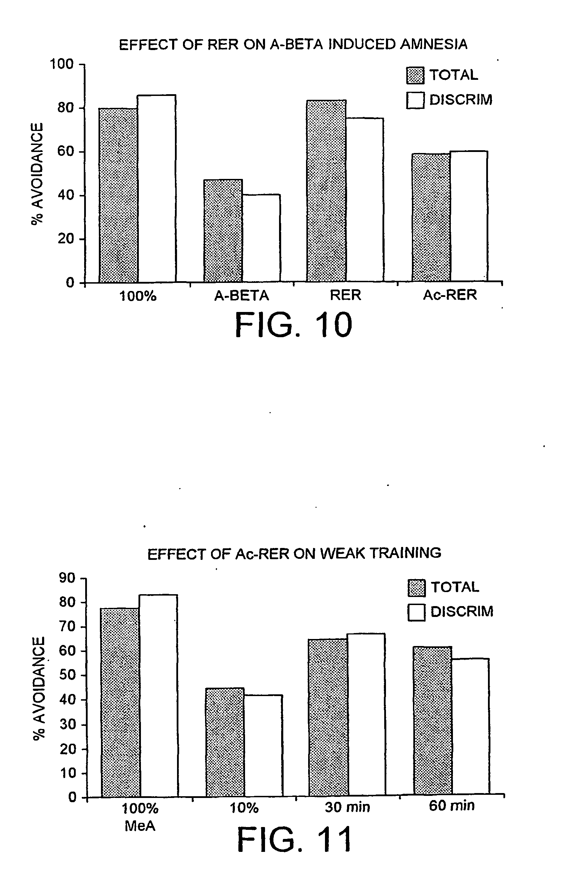

Figure 10 shows the effect of RER and Ac-RER on β-amyloid 12-28 induced amnesia in chicks trained on the "strong" memory test described hereinbefore.

Figure 10 shows from left to right the percent avoidance measured in terms of total avoidance and discrimination for
- chicks trained on the "strong" version of the training;
- the amnesic effect of β-amyloid 12-28 administered at 2 µg/hemisphere 30 mins prior to training;
- the effect of RER administered at 2 µg/hemisphere 20 mins prior to training after administration of β-amyloid 12-28 30 mins prior to training; and
- the effect of Ac-RER administered at 2 µg/hemisphere 20 mins prior to training after administration of β-amyloid 12-28 30 mins prior to training.

The results in figure 10 show that both RER and Ac-RER have the effect of restoring memory measured 20 mins after administration of RER or Ac-RER, following amnesia induced by administration of β-amyloid 12-28.

The results also show that the memory-restorative effect of Ac-RER is less than that of RER but indicate that Ac-RER is more stable after administration and therefore more suitable for peripheral administration.

Figure 11 shows the effect of Ac-RER on memory in chicks trained on the "weak" memory test described hereinbefore.

Figure 11 shows from left to right the percent avoidance measured in terms of total avoidance and discrimination for
- chicks trained on the "strong" version of the training:
- chicks trained on the "weak" version of the training;
- chicks trained on the "weak" version of the training after having had Ac-RER administered at 2 µg/hemisphere 30 mins beforehand; and
- chicks trained on the "weak" version of the training after having had Ac-RER administered at 2 µg/hemisphere 60 mins beforehand.

The results in figure 11 show that Ac-RER has the effect of improving the memory of weakly trained chicks when administered 30 or 60 minutes beforehand. The results also show that the effect 60 minutes after administration is still significant, although less than 30 minutes after administration.

The results therefore show that Ac-RER has the effect of a cognitive enhancer and that the effect is apparent over a significant time period.

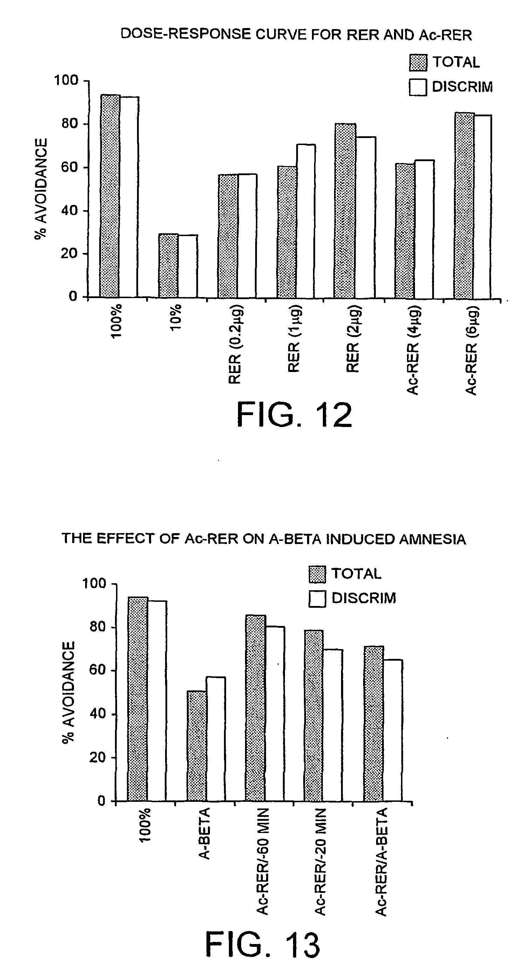

Figure 12 shows the effect of different doses of RER and Ac-RER on memory in chicks trained on the "weak" memory test described hereinbefore.

Figure 12 shows from left to right the percent avoidance measured in terms of total avoidance and discrimination for
- chicks trained on the "strong" version of the training;
- chicks trained on the "weak" version of the training;
- chicks trained on the "weak" version of the training after having had RER administered 30 mins beforehand at 0.2 µg/hemisphere;
- chicks trained on the "weak" version of the training after having had RER administered 30 mins beforehand at 1.0 µg/hemisphere;
- chicks trained on the "weak" version of the training after having had RER administered 30 mins beforehand at 2.0 µg/hemisphere;
- chicks trained on the "weak" version of the training after having had Ac-RER administered 30 mins beforehand at 4.0 µg/hemisphere; and
- chicks trained on the "weak" version of the training after having had Ac-RER administered 30 mins beforehand at 6.0 µg/hemisphere.

The results in figure 12 show that both RER and Ac-RER have the effect of improving the memory of weakly-trained chicks when administered 30 minutes beforehand and that higher doses of Ac-RER than of RER are required to obtain a comparable effect.

The results therefore show that increased amounts of Ac-RER, in comparison to RER, are required to be used as a cognitive enhancer. This again indicates that Ac-RER is more stable after administration than RER and therefore more suitable for peripheral administration.

Figure 13 shows the effect of Ac-RER administered at different times on β-amyloid 12-28 induced amnesia in chicks trained on the "strong" memory test described hereinbefore.

Figure 13 shows from left to right the percent avoidance measured in terms of total avoidance and discrimination for
- chicks trained on the "strong" version of the training;
- the amnesic effect of β-amyloid 12-28 administered at 2 µg/hemisphere 30 mins prior to training;
- the memory-restorative effect of Ac-RER administered at 4 µg/hemisphere 60 mins prior to training and 30 mins prior to administration of β-amyloid 12-28 at 2 µg/hemisphere;
- the memory-restorative effect of Ac-RER administered at 4 µg/hemisphere 20 mins prior to training and 10 mins after administration of β-amyloid 12-28 at 2 µg/hemisphere; and
- the memory-restorative effect of Ac-RER administered at 4 µg/hemisphere together with β-amyloid 12-28 at at 2 µg/hemisphere 30 mins prior to training.

The results of figure 13 show that Ac-RER has the effect of restoring memory when amnesia has been induced by β-amyloid 12-28. The effect is manifested regardless of whether Ac-RER is administered before, after or simultaneously with the amnesia-inducing β-amyloid 12-28.

The role of APP in memory formation has been attributed to its involvement in cell-to-substrate adhesion processes. The data reported suggests that the APP involvement in memory formation most probably involves change in signal transduction events. The post-training time within which the antibody and antisense-induced amnesia, and within which RER, Ac-RER, RERMS and SMRER prevents amnesia, corresponds to that during which memory formation is vulnerable to disruption of the putative signal-transduction functions of APP.

The chick system is a good one for exploring these issues, because the learning task is precise and sharply timed, and permits one also to be sure that any observed effect of an injected substance is specific to retention and not either to acquisition or to concomitant processes such as visual acuity, arousal or motor activity. Further, the role of other cell adhesion molecules in the cascade leading to synaptic modulation has been well mapped, so that the effects of either blocking or attempting to rescue functional APP activity can be set into an established context : see Rose, Learn. Memory 7, 1-17 (2000) .

It is therefore indicated by the experimental results reported above that compounds of the present invention are effective for the treatment and/or prevention of neurological diseases and disorders and as cognitive enhancers (nootropic agents) in other animals, including human and non-human mammals. The compounds are therefore effective in the treatment and/or prevention of Alzheimer's disease in humans and other neurodegenerative diseases and disorders in animals generally, including humans. Such animals include transgenic and other animal models for Alzheimer's disease.

As used herein, except where the context indicates otherwise, the terms "treatment", "treat" and analogous expressions used in relation to neurodegenerative diseases include within their scope not only treatment when symptoms are apparent but also the partial or total prevention of such diseases and delay in their onset in patients or animals who are subjected to treatment before onset of the disease or its symptoms become apparent.

The compounds of the present invention may be administered intracerebally as described above, or may be administered peripherally, for example intramuscularly, intravenously, transdermally or orally, preferably after complexation as described above. Instead or in addition, the compounds may be protected against alteration between administration and effectiveness, for example by addition of protective groups.

The experimental results of figures 10 to 13 show that the polypeptide derivatives of the present invention, particularly Ac-RER, are effective for longer time periods after administration than their analogues lacking protective groups and are therefore particularly suitable for peripheral administration.

The compounds of the present invention may also be formulated as pharmaceutical compositions as referred to hereinbefore, particularly such compositions as are capable of crossing the blood-brain barrier and thereby be suitable for peripheral administration.

In all events a suitable dose of peptide compounds according to the invention is from 10 to 100 µg/kg body weight of the animal being treated.

As used herein, the term "effective to treat" in the context of a neurodegenerative disease means that amount of the compound(s) used in the treatment which causes a reduction or stabilisation or, as the case may be, prevents or delays the appearance of such symptoms as measured by standard medical or psychological criteria, for example as disclosed in Handbook of Memory Disorders (eds: A D Baddeley, B A Wilson and F N Watts), Wiley (1995), .

As used herein, the term "effective to treat" in relation to a cognitive enhancement means an amount of the compound(s) used in the treatment which causes an improvement in cognitive power as measured by psychological criteria, for example as disclosed in Handbook of Memory Disorders (eds: A D Baddeley, B A Wilson and F N Watts), Wiley (1995), .

### SEQUENCE LISTING FREE TEXT

| SEQ ID NO. | Free Text <223> |
|---|---|
| 3 | Description of Artificial Sequence: 5-mer polypeptide |
| 4 | Description of Artificial Sequence: 5-mer polypeptide |
| 5 | Description of Artificial Sequence: 5-mer polypeptide |
| 6 | Description of Artificial Sequence: 17-mer polypeptide |
| 7 | Description of Artificial Sequence: 17-mer polypeptide |
| 8 | Description of Artificial Sequence: 17-mer polypeptide |
| 9 | Description of Artificial Sequence: 3-mer polypeptide |
| 10 | Description of Artificial Sequence: 4-mer polypeptide |
| 11 | Description of Artificial Sequence: 4-mer polypeptide |
| 12 | Description of Artificial Sequence: 16-mer polypeptide |
| 13 | Description of Artificial Sequence: 15-mer polypeptide |
| 14 | Description of Artificial Sequence: 14-mer polypeptide |
| 15 | Description of Artificial Sequence: 12-mer polypeptide |
| 16 | Description of Artificial Sequence: 11-mer polypeptide |
| 17 | Description of Artificial Sequence: 11-mer polypeptide |
| 18 | Description of Artificial Sequence: 10-mer polypeptide |
| 19 | Description of Artificial Sequence: 6-mer polypeptide |
| 20 | Description of Artificial Sequence: 22-mer polypeptide |
| 21 | Description of Artificial Sequence: end-protected 16-mer oligonucleotide modified at positions 2 & 13 to prevent internal looping |
| 22 | Description of Artificial Sequence: 40-mer polypeptide |

## Claims

1. A pharmaceutical composition comprising: (a) a compound having the formula: 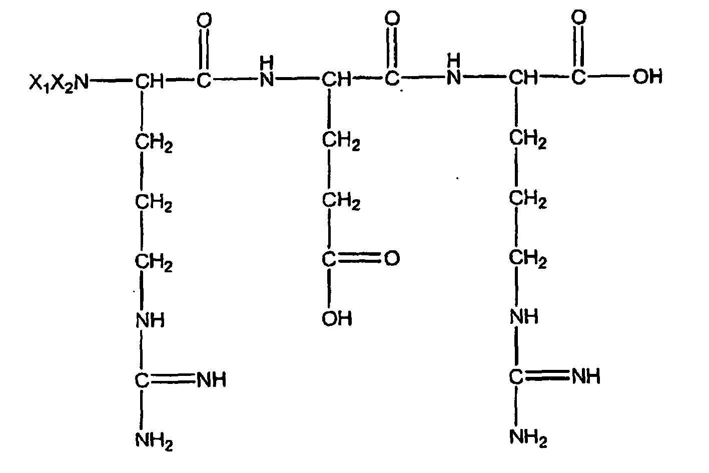 in which X₁ is an acyl group represented by the formula: 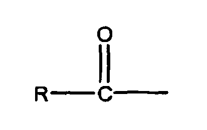 X₂ is H or an acyl group represented by the formula: 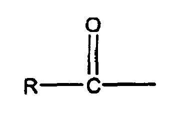 and R represents a straight- or branched-chain alkyl group, an alkyl-substituted or unsubstituted cycloalkyl group, a straight or branched-chain aralkyl group or an alkyl-substituted or unsubstituted aryl group, the groups R being the same or different when two are present, and (b) a pharmaceutically acceptable carrier.

2. A pharmaceutical composition according to claim 1, wherein X₂ is H.

3. A pharmaceutical composition according to claim 2, in which R represents a C₁ to C₆ straight- or branched-chain alkyl group.

4. A pharmaceutical composition according to claim 3, in which R is a methyl group.

5. A pharmaceutical composition according to any of claims 1 to 4, in which the compound is physically linked to a further molecule or vehicle for pharmaceutical delivery of the compound.

6. A compound having the formula: 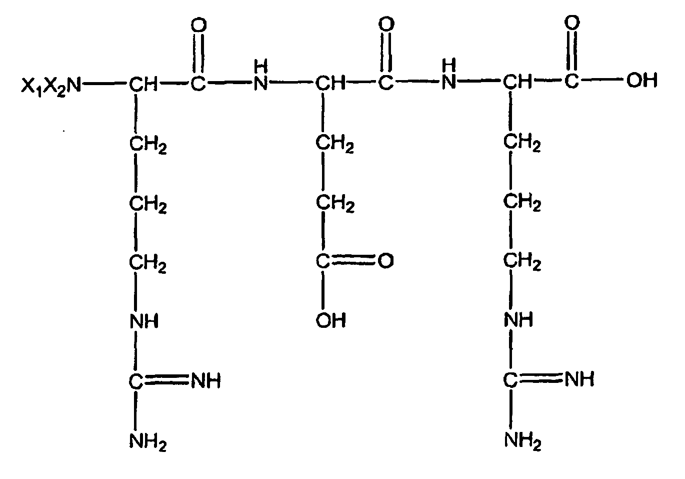 in which X₁ is an acyl group represented by the formula: 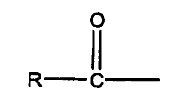 X₂ is H or an acyl group represented by the formula: 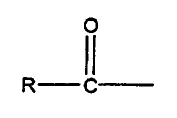 and R represents a straight- or branched-chain alkyl group, an alkyl-substituted or unsubstituted cycloalkyl group, a straight or branched-chain aralkyl group or an alkyl-substituted or unsubstituted aryl group, the groups R being the same or different when two are present, for use in medicine.

7. A compound according to claim 6, wherein X₂ is H.

8. A compound according to claim 7, in which R represents a C₁ to C₆ straight- or branched-chain alkyl group.

9. A compound according to claim 8, in which R is a methyl group.

10. A compound according to any of claims 6 to 9, which is physically linked to a further molecule or vehicle for pharmaceutical delivery of the compound.

11. A compound according to any of claims 6 to 10, for use in the treatment of a neurodegenerative disease.

12. A compound according to any of claims 6 to 10, for use in the treatment of Alzheimer's disease.

13. Use of a compound as defined in any of claims 6 to 10, in the preparation of a medicament for use in the treatment of a neurodegenerative disease.

14. Use of a compound as defined in any of claims 6 to 10, in the preparation of a medicament for use in the treatment of Alzheimer's disease.

15. A compound according to any of claims 6 to 10, for use as a cognitive enhancer.

16. Use of a compound as defined in any of claims 6 to 10, in the preparation of a medicament for use as a cognitive enhancer.

17. A non-therapeutic, non-surgical method of producing a cognitive enhancement effect in an animal, comprising
providing an animal in which the effect is to be produced; and
administering to the animal an amount of a compound effective to produce the cognitive enhancement, the compound having the formula: 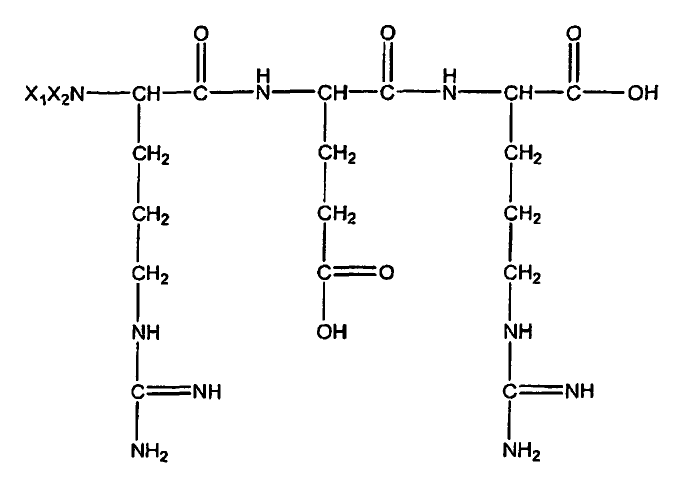 in which X₁ is an acyl group represented by the formula: 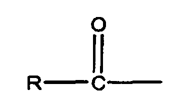 X₂ is H or an acyl group represented by the formula: 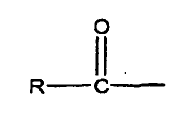 and R represents a straight- or branched-chain alkyl group, an alkyl-substituted or unsubstituted cycloalkyl group, a straight or branched-chain aralkyl group or an alkyl-substituted or unsubstituted aryl group, the groups R being the same or different when two are present.

18. A method according to claim 17, wherein X₂ is H.

19. A method according to claim 18, in which R represents a C₁ to C₆ straight- or branched-chain alkyl group.

20. A method according to claim 19, in which R is a methyl group.

21. A method according to any of claims 17 to 20, in which the said compound is administered in the form of a pharmaceutical composition comprising said compound and a pharmaceutically acceptable carrier.

22. A method according to any of claims 17 to 20, in which the said compound is physically linked to a further molecule or vehicle for pharmaceutical delivery of the compound.

23. A method according to any of claims 17 to 22, in which the animal is a human.

## Patentansprüche

1. Pharmazeutische Zusammensetzung, die Folgendes umfasst: (a) eine Verbindung mit der Formel: 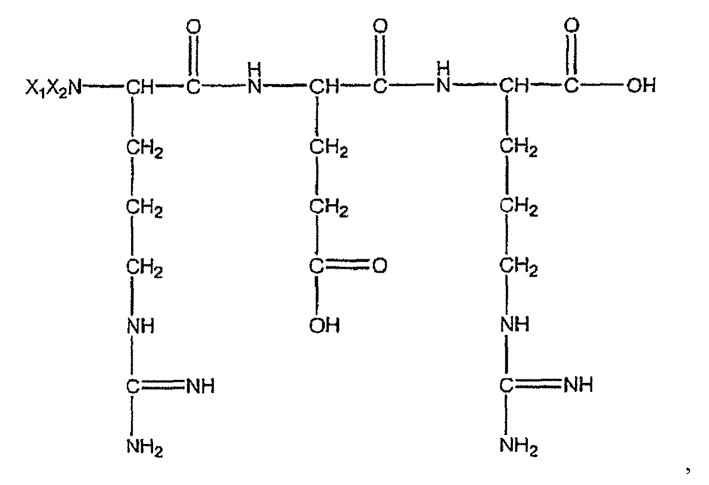 in der X₁ eine Acylgruppe ist, die durch die folgende Formel dargestellt wird: 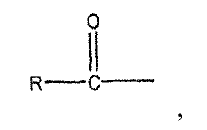 X₂ H oder eine Acylgruppe ist, die durch die folgende Formel dargestellt wird: 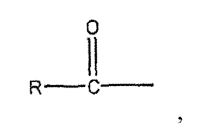 und R eine gerad- oder verzweigtkettige Alkylgruppe, eine Alkyl-substituierte oder unsubstituierte Cycloalkylgruppe, eine gerad- oder verzweigtkettige Aralkylgruppe oder eine Alkyl-substituierte oder unsubstituierte Arylgruppe darstellt, wobei die Gruppen R gleich oder verschieden sind, wenn zwei vorliegen, und
(b) einen pharmazeutisch verträglichen Träger.

2. Pharmazeutische Zusammensetzung nach Anspruch 1, worin X₂ H ist.

3. Pharmazeutische Zusammensetzung nach Anspruch 2, in der R eine C₁- bis C₆-gerad- oder -verzweigtkettige Alkylgruppe darstellt.

4. Pharmazeutische Zusammensetzung nach Anspruch 3, in der R eine Methylgruppe ist.

5. Pharmazeutische Zusammensetzung nach einem der Ansprüche 1 bis 4, in der die Verbindung mit einem weiteren Molekül oder Vehikel zur pharmazeutischen Abgabe der Verbindung physisch verbunden ist.

6. Verbindung mit der Formel: 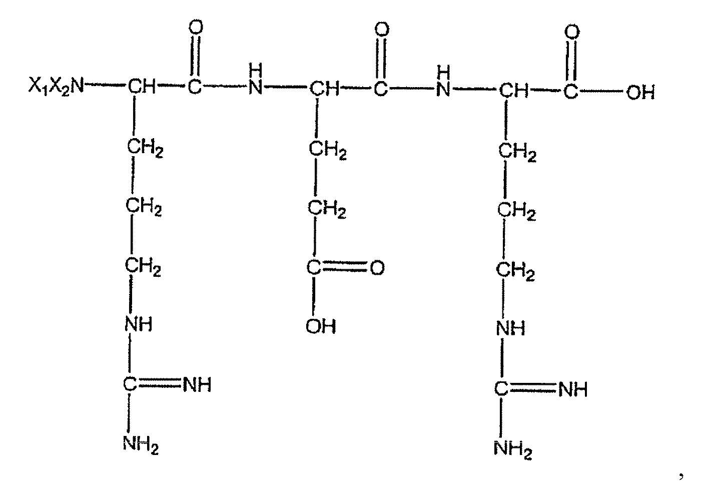 in der X₁ eine Acylgruppe ist, die durch die folgende Formel dargestellt wird: 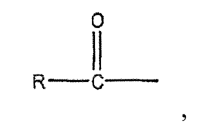 X₂ H oder eine Acylgruppe ist, die durch die folgende Formel dargestellt wird: 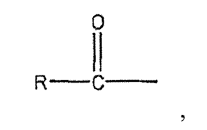 und R eine gerad- oder verzweigtkettige Alkylgruppe, eine Alkyl-substituierte oder unsubstituierte Cycloalkylgruppe, eine gerad- oder verzweigtkettige Aralkylgruppe oder eine Alkyl-substituierte oder unsubstituierte Arylgruppe darstellt, wobei die Gruppen R gleich oder verschieden sind, wenn zwei vorliegen, zur Verwendung in der Medizin.

7. Verbindung nach Anspruch 6, worin X₂ H ist.

8. Verbindung nach Anspruch 7, in der R eine C₁- bis C₆-gerad- oder -verzweigtkettige Alkylgruppe darstellt.

9. Verbindung nach Anspruch 8, in der R eine Methylgruppe ist.

10. Verbindung nach einem der Ansprüche 6 bis 9, die mit einem weiteren Molekül oder Vehikel zur pharmazeutischen Abgabe der Verbindung physisch verbunden ist.

11. Verbindung nach einem der Ansprüche 6 bis 10 zur Verwendung bei der Behandlung einer neurodegenerativen Erkrankung.

12. Verbindung nach einem der Ansprüche 6 bis 10 zur Verwendung bei der Behandlung von Alzheimer-Erkrankung.

13. Verwendung einer Verbindung, wie in einem der Ansprüche 6 bis 10 definiert, bei der Herstellung eines Medikaments zur Verwendung bei der Behandlung einer neurodegenerativen Erkrankung.

14. Verwendung einer Verbindung, wie in einem der Ansprüche 6 bis 10 definiert, bei der Herstellung eines Medikaments zur Verwendung bei der Behandlung von Alzheimer-Erkrankung.

15. Verbindung nach einem der Ansprüche 6 bis 10 zur Verwendung als kognitiver Verstärker.

16. Verwendung einer Verbindung, wie in einem der Ansprüche 6 bis 10 definiert, bei der Herstellung eines Medikaments zur Verwendung als kognitiver Verstärker.

17. Nichttherapeutisches, nichtoperatives Verfahren zur Erzeugung einer Wirkung der kognitiven Verstärkung bei einem Tier, das Folgendes umfasst:
Bereitstellen eines Tiers, in dem die Wirkung zu erzeugen ist; und
Verabreichen einer Menge einer Verbindung, die zur Erzeugung der kognitiven Verstärkung wirksam ist, an das Tier, wobei die Verbindung die folgende Formel hat: 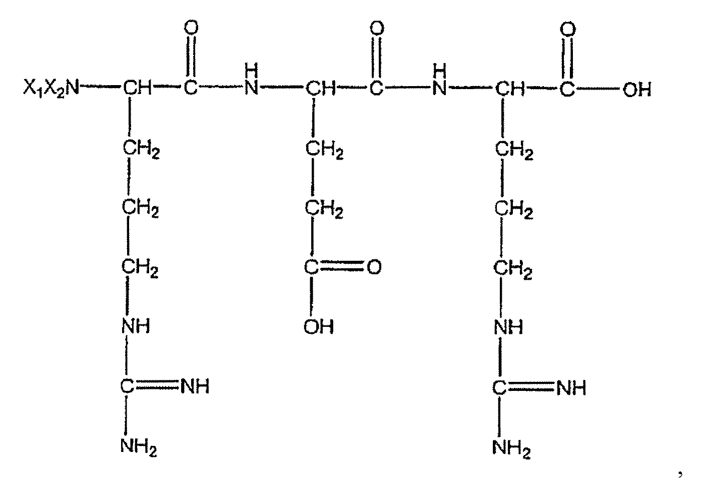 in der X₁ eine Acylgruppe ist, die durch die folgende Formel dargestellt wird: 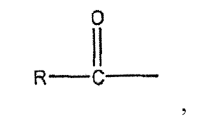 X₂ H oder eine Acylgruppe ist, die durch die folgende Formel dargestellt wird: 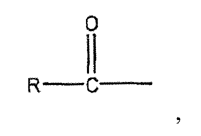
und R eine gerad- oder verzweigtkettige Alkylgruppe, eine Alkyl-substituierte oder unsubstituierte Cycloalkylgruppe, eine gerad- oder verzweigtkettige Aralkylgruppe oder eine Alkyl-substituierte oder unsubstituierte Arylgruppe darstellt, wobei die Gruppen R gleich oder verschieden sind, wenn zwei vorliegen.

18. Verfahren nach Anspruch 17, worin X₂ H ist.

19. Verfahren nach Anspruch 18, in dem R eine C₁- bis C₆-gerad- oder -verzweigtkettige Alkylgruppe darstellt.

20. Verfahren nach Anspruch 19, in dem R eine Methylgruppe ist.

21. Verfahren nach einem der Ansprüche 17 bis 20, in dem die genannte Verbindung in Form einer pharmazeutischen Zusammensetzung, die die genannte Verbindung und einen pharmazeutisch verträglichen Träger umfasst, verabreicht wird.

22. Verfahren nach einem der Ansprüche 17 bis 20, in dem die genannte Verbindung mit einem weiteren Molekül oder Vehikel zur pharmazeutischen Abgabe der Verbindung physisch verbunden ist.

23. Verfahren nach einem der Ansprüche 17 bis 20, in dem das Tier ein Mensch ist.

## Revendications

1. Composition pharmaceutique comprenant : (a) un composé de formule : 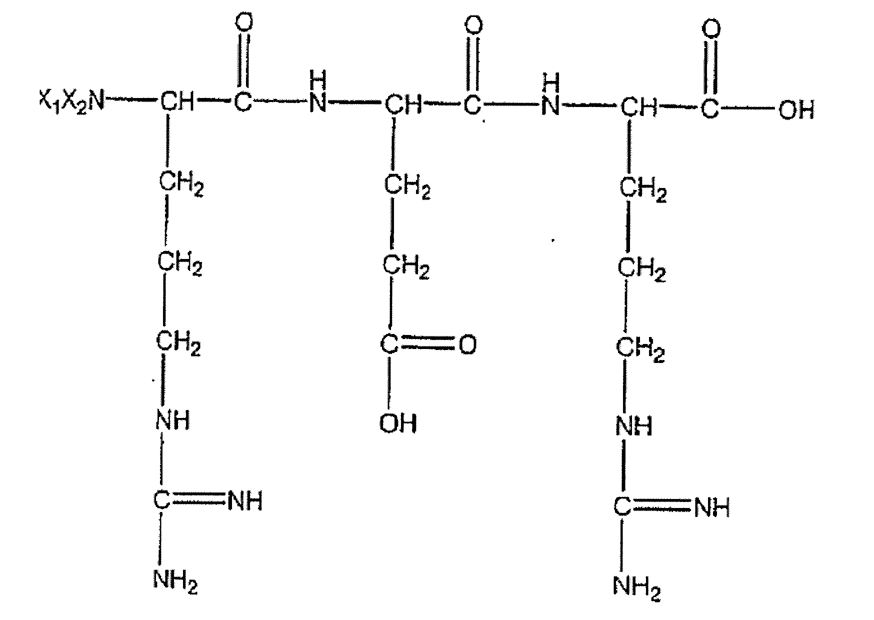 dans laquelle X₁ est un groupement acyle représenté par la formule : 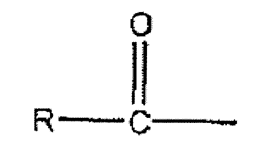 X₂ est H ou un groupement acyle représenté par la formule : 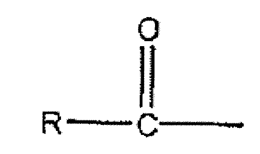 et R représente un groupement alkyle à chaîne droite ou ramifiée, un groupement cycloalkyle substitué par un alkyle ou non substitué, un groupement aralkyle à chaîne droite ou ramifiée ou un groupement aryle substitué par un alkyle ou non substitué, les groupements R étant identiques ou différents lorsque deux sont présents et (b) un support pharmaceutiquement acceptable.

2. Composition pharmaceutique selon la revendication 1, dans laquelle X₂ est H.

3. Composition pharmaceutique selon la revendication 2, dans laquelle R représente un groupement alkyle en C₁ à C₆ à chaîne droite ou ramifiée.

4. Composition pharmaceutique selon la revendication 3, dans laquelle R est un groupement méthyle.

5. Composition pharmaceutique selon l'une quelconque des revendications 1 à 4, dans laquelle le composé est physiquement lié à une molécule ou un véhicule supplémentaire pour l'administration pharmaceutique du composé.

6. Composé de formule : 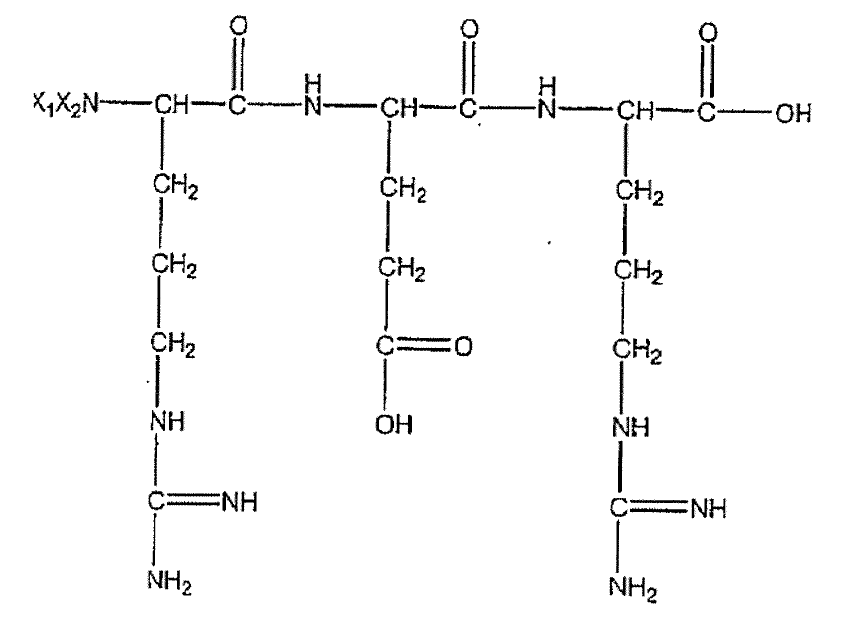 dans laquelle X₁ est un groupement acyle représenté par la formule : 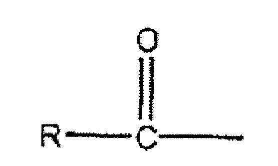 X₂ est H ou un groupement acyle représenté par la formule : 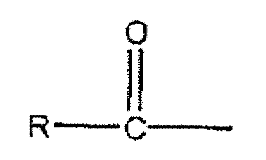 et R représente un groupement alkyle à chaîne droite ou ramifiée, un groupement cycloalkyle substitué par un alkyle ou non substitué, un groupement aralkyle à chaîne droite ou ramifiée ou un groupement aryle substitué par un alkyle ou non substitué, les groupements R étant identiques ou différents lorsque deux sont présents, destiné à être utilisé en médecine.

7. Composé selon la revendication 6, dans lequel X₂ est H.

8. Composé selon la revendication 7, dans lequel R représente un groupement alkyle en C₁ à C₆ à chaîne droite ou ramifiée.

9. Composé selon la revendication 8, dans lequel R est un groupement méthyle.

10. Composé selon l'une quelconque des revendications 6 à 9, le composé étant physiquement lié à une molécule ou un véhicule supplémentaire pour l'administration pharmaceutique du composé.

11. Composé selon l'une quelconque des revendications 6 à 10, destiné à être utilisé dans le traitement d'une maladie neurodégénérative.

12. Composé selon l'une quelconque des revendications 6 à 10, destiné à être utilisé dans le traitement de la maladie d'Alzheimer.

13. Utilisation d'un composé tel que défini dans l'une quelconque des revendications 6 à 10, pour la préparation d'un médicament destiné à être utilisé dans le traitement d'une maladie neurodégénérative.

14. Utilisation d'un composé tel que défini dans l'une quelconque des revendications 6 à 10, pour la préparation d'un médicament destiné à être utilisé dans le traitement de la maladie d'Alzheimer.

15. Composé selon l'une quelconque des revendications 6 à 10, destiné à être utilisé en tant qu'agent améliorant la fonction cognitive.

16. Utilisation d'un composé tel que défini dans l'une quelconque des revendications 6 à 10, pour la préparation d'un médicament destiné à être utilisé en tant qu'agent améliorant la fonction cognitive.

17. Procédé non thérapeutique, non chirurgical de production d'un effet améliorant la fonction cognitive chez un animal, comprenant les étapes consistant à
fournir un animal chez lequel l'effet est à produire ; et
administrer à l'animal une quantité d'un composé efficace pour produire l'amélioration de la fonction cognitive, le composé ayant la formule : 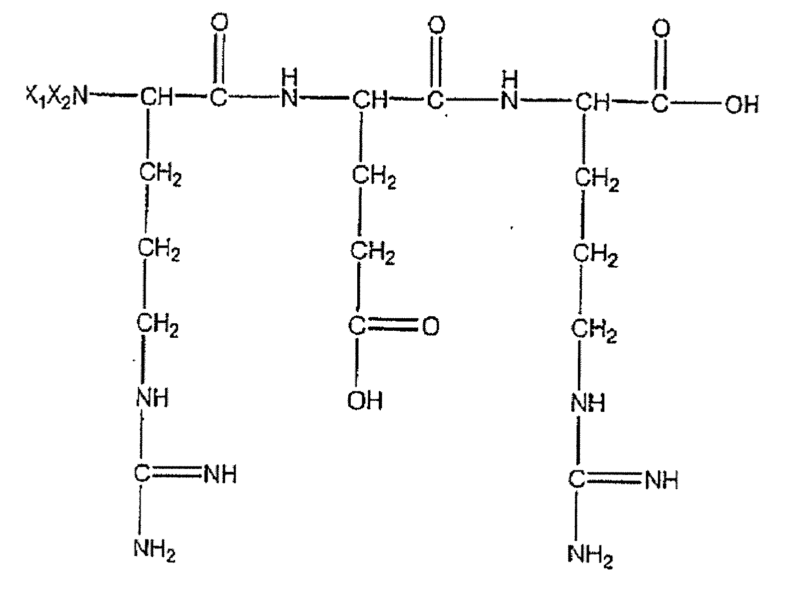 dans laquelle X₁ est un groupement acyle représenté par la formule : 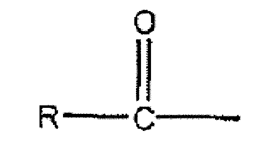 X₂ est H ou un groupement acyle représenté par la formule : 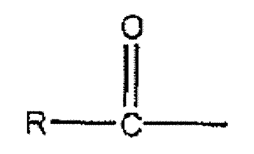 et R représente un groupement alkyle à chaîne droite ou ramifiée, un groupement cycloalkyle substitué par un alkyle ou non substitué, un groupement aralkyle à chaîne droite ou ramifiée ou un groupement aryle substitué par un alkyle ou non substitué, les groupements R étant identiques ou différents lorsque deux sont présents.

18. Procédé selon la revendication 17, dans lequel X₂ est H.

19. Procédé selon la revendication 18, dans lequel R représente un groupement alkyle en C₁ à C₆ à chaîne droite ou ramifiée.

20. Procédé selon la revendication 19, dans lequel R est un groupement méthyle.

21. Procédé selon l'une quelconque des revendications 17 à 20, ledit composé étant administré sous forme d'une composition pharmaceutique comprenant ledit composé et un support pharmaceutiquement acceptable.

22. Procédé selon l'une quelconque des revendications 17 à 20, ledit composé étant physiquement lié à une molécule ou un véhicule supplémentaire pour l'administration pharmaceutique du composé.

23. Procédé selon l'une quelconque des revendications 17 à 22, dans lequel l'animal est un être humain.
